# EUROPEAN PATENT APPLICATION

(11) **EP 2 469 432 A1**
(43) Date of publication of application: **27.06.2012**
(21) Application number: 10016077.9
(22) Date of filing: 24.12.2010
(51) Int. Cl.: G06F 19/00

(54) **Ambulatory infusion system with alert prioritizing features**

(71) Applicant: F.Hoffmann-La Roche AG, 4070 Basel (CH); Roche Diagnostics GmbH, 68305 Mannheim (DE)
(72) Inventor: Bernini, Nicole, 3400 Burgdorf (CH); Sigrist, Reto, 3207 Golaten (CH); Schütz, Andrea, 4150 Schwarzenburg (CH)
(74) Representative: Rentsch Partner AG

(57) **Abstract**

Disclosed is
an ambulatory infusion system (100, 100', 600), the ambulatory infusion system (100, 100', 600), including:
(a) an electronically controlled dosing unit (110), the dosing unit (110) being designed to be carried by a user for an extended time period and concealed from view,
(b) a monitoring unit (111, 120, 620), the monitoring unit (111, 120, 620) being designed to repeatedly test the occurrence of a number of alerting conditions of the ambulatory infusion system (100, 100', 620) and to change, upon detection of an alerting condition, a corresponding alerting status from a deactivated state to an activated state while maintaining other alerting statuses unaffected,
(c) an alerting unit (130, 630), the alerting unit (130, 630) being designed to provide an alert to a device user with respect to an alerting condition, the alert including information identifying the alerting condition;
(d) an alert handling unit (121, 621), the alert handling unit (121, 621) storing an alerting priority for each of the alerting conditions and being designed to repeatedly determine, among the activated alerting statuses, an alerting status of the highest alerting priority and to control the alerting unit (130, 630) to provide an alert with respect to the corresponding alerting condition.

## Description

### Technical Field

The present disclosure is related to the technical field of ambulatory infusion systems including an electronically controlled dosing unit.

### Background

Ambulatory infusion systems are known in the art for a variety of applications. In particular, systems adapted for insulin administration form a basis for the state-of-the-art therapy of diabetes mellitus by CSII (Continuous Subcutaneous Insulin Infusion). The systems are typically based on computer-controlled micro-dosing pumps that are adapted to be worn continuously and concealed from view, e.g., in a clothes pocket, with a belt clip, or as patch that is adhesively attached to the skin. The insulin is infused via a subcutaneous cannula that is replaced by a user, e.g. a PwD (Person with Diabetes) or a relative of such a person, every few days. Such insulin pumps are commercially available from a number of suppliers.

Insulin pumps are typically designed to infuse a liquid insulin formulation substantially continuously according to a basal administration profile that is variable over the time of day. In addition, they are typically designed to infuse larger drug boli in a comparatively short time interval on demand. In CSII, insulin boli are typically administered to compensate food intake and to lower an undesirably raised blood glucose level. The total daily amount of infused insulin may vary in dependence on personal factors and habits of a PwD in a considerable range (between, e.g., 10IU to 80IU, with 100IU (International units) corresponding to 1ml of a liquid insulin formulation for the currently most common concentration U100).

Those infusion systems often include a positive-displacement pump of the syringe-driver type that is, during application, coupled to a cylindrical drug cartridge out of which a liquid drug formulation is forced into an infusion line by displacing a cartridge plunger in a controlled manner. Reference is made to the disclosures of the WO 2003053498 A2 and the WO2000025844A1 for exemplary typical designs and typical features of a state-of-the-art infusion pump. The present disclosure, however, is not limited to a specific therapy or system design.

In the following, reference is mainly made to CSII as exemplary field of application. Phrases like "infusion system" or "infusion device" may therefore refer to systems and devices which are suitable for CSII. Besides CSII, those systems and devices may also be used in similar applications, such as various hormone therapies, pain therapy or cancer treatment without requiring substantive modification, if any.

### Summary of Disclosure

Typical ambulatory infusion systems are designed to automatically detect the presence of a number of alerting conditions and to provide corresponding alerts to a device user.

In the context of the present disclosure, the term "user" typically refers to a person that carries the ambulatory infusion system for therapeutic purposes, such as a PwD. It may, however, also refer to another person that operates the device, such as a parent of a diabetic child, a relative, or the like. The user is typically trained with respect to everyday handling of the system, replacing disposables, and the like, but is typically not a medical professional nor a device expert.

Alerting conditions may occur for a number of reasons. They may, for example, occur due to the occurrence of a fluidic hazard, such as a blockage of an infusion tubing or an infusion cannula, a leakage, or the like. Further typical alerting conditions are the occurrence of technical problems, such as mechanical or electronic device defectives. Further typical alerting conditions are associated with an upcoming or immediately required replacement of a disposable, such as a drug cartridge which has typically to be replaced every few days, or of a battery powering the system, which may have a lifetime in a range of some days up to several months. Still further alerting conditions may be associated with user reminders, such as a general alarm clock function, a reminder to replace an infusion tubing and/or infusion cannula, which has typically replaced by the user every few days in order to prevent blockages, to prevent inflammation, and the like.

With the system complexity and the safety requirements steadily increasing, the number of potential alerting conditions, and consequently, the overall number of alerts increases, too. This situation is further added to by ambulatory infusion systems consisting more and more of a number of individual devices, such as a dosing device and a separate remote control device which may also include an analyte measuring device, such as a blood glucose measurement device. Furthermore, such a system may include or operatively couple to a continuous analyte measuring system, such as a continuous blood glucose measuring system. A number of alerting conditions typically exists for each of those individual devices. The WO2010017886A1 discloses an ambulatory infusion system that allows time synchronizing alerts with respect to different alert reasons, thus reducing the total number of alerts at different times.

Due to the generally increasing number of different alerting conditions, there is an increasing need for handling alerting conditions and providing alerts in appropriate way, taking into account the fact that a number of alerting conditions may occur within a short time period and the urgency for a system user to react onto an alerting condition may be very different for different alerting conditions.

The problem is solved based on the insight that it is favorable to decouple the detecting of an alerting condition from providing a corresponding alert, based on an alerting priority that is assigned to each alerting condition. That is, an alert is not necessarily provided immediately upon occurrence of the corresponding alerting condition but can be provided at a later point in time when no alerting condition of higher alerting priority is present. In this way, all alerting conditions can be indicted to the user, but alerts of higher urgency, and, accordingly, higher the priority, are provided first, even if the alerting condition occurred at a later point in time. Generally, important and/or critical alerts, and in particular alerts that require immediate notification and/or action, have a higher alerting priority as compared to alerts that allow more time to react on or are provided for information purposes only.

An ambulatory infusion system in accordance with the present disclosure may include an electronically controlled dosing unit, the dosing unit being designed to be carried by a user for an extended time period and concealed from view. The dosing unit may, for example, include a typically motor-driven spindle drive, a micro membrane pump, a peristaltic pump, a micro piston pump, such as a dosing unit as disclosed in the EP1970677A1 and the EP2163273A1, or the like. The dosing unit may be controlled to administer drug in accordance with a time-variable basal administration profile in a continuous or quasi-continuous way and to further administer larger drug boli on demand.

The ambulatory infusion system may include a monitoring unit, the monitoring unit be designed to repeatedly test the occurrence of a number of alerting conditions and to change, upon detection of an alerting condition, a corresponding alerting status from an deactivated state to an activated state, while maintaining other alerting statuses unaffected. In the context of the present disclosure, changing an alerting status from its deactivated state to its activated state is referred to as "activating the alerting status". Similarly, changing an alerting status from its activated state to its deactivated state is referred to as "deactivating the alerting status".

The monitoring unit may include a sensor unit, the sensor unit being operatively coupled to the dosing unit to monitor a physical characteristic, the physical characteristic reflecting an occurrence of an alerting condition of the dosing unit. A sensor unit may include one or multiple distinct sensors. For a syringe-driver type system, the sensor unit may, for example, include a force sensor that measures the reaction force that is exerted onto a syringe plunger and/or a rotary encoder that monitors operation of a typically present drive motor. Additional or alternative sensors, such as a fluidic pressure sensor or a fluidic flow sensor or an air-bubble sensor, may be present as well.

An alerting status, once activated, stays activated until it is explicitly deactivated as will be discussed below. The mechanism of individually activating and deactivating alerting statuses realizes a temporary storing of activated alerting conditions until they are deactivated. Accordingly, the activation of an alerting status is not "lost" even if the alert indication is not provided immediately because of the presence of other alerting conditions with higher alerting priority.

The ambulatory infusion system may include an alerting unit, the alerting unit being designed to provide an alert to a device user with respect to an alerting cause. The alert includes information identifying the alerting condition.

The ambulatory infusion system may include an alert handling unit, the alert handling unit storing an alerting priority for each of the alerting conditions and being designed to repeatedly determine, among the activated alerting statuses, an alerting status of the highest alerting priority and to control the alerting unit to provide an alert with respect to the corresponding alerting condition.

The ambulatory infusion system may be realized as a single physical unit which may be designed to also receive a drug cartridge and one or multiple batteries, or may be split into a number of distinct physical units, such as a disposable unit and a reusable unit, wherein the disposable unit is intended for a single usage time, of typically a few days of up two years, whereas the reusable unit is typically designed for a lifetime of several months of up to some years. The system may alternatively be fully disposable. The system may further include additional further separate devices, such as a remote control device

The alert handling unit and the monitoring unit may, fully or partly, be realized in an integral way with general control circuitry of the ambulatory infusion system and be realized, fully or partly, as firmware running on one or multiple microcontrollers, ASICS (Application Specific Integrated Circuits), conventional semiconductor-based circuitry, or any combination of those.

An ambulatory infusion system in accordance with the present disclosure allows, once an alerting condition has occurred, to temporarily store the corresponding alerting status and until a corresponding alert is provided and subsequently confirmed. Storing a priority for each alerting condition ensures that alerts are provided in an appropriate order. In particular, it ensures that an alert having a higher alerting priority is given prior to an alert having a lower alerting priority, even if the corresponding alerting condition occurred later.

An alerting status may generally be considered as a binary flag that is set upon the corresponding alerting status being activated and is reset upon the alerting status being deactivated. Various specific ways of storing activated alerting statuses and determining an alerting status for the alert generation will be discussed in more detail in the context of various embodiments.

In some embodiments, the ambulatory infusion system is designed to store at least three different alerting priorities. At least three different alerting priorities are believed to be appropriate in many cases. For example, a highest priority may be provided for system defects and alert causes that require substantially immediate action, such as a blocked cannula. A medium priority may be used for alert causes that warn the with respect to an upcoming user action in the nearer future, such as a replacement of the drug cartridge. A lowest priority level may be used for alert causes that require no user action, such as pure reminders. However, a different number of alerting priorities, in particular a higher number, may be provided as well.

The alerting conditions may include:
- at least one malfunction alerting condition, the at least one malfunction alerting condition being indicative for a malfunction of the ambulatory infusion system, the at least one malfunction alerting condition having a high alerting priority,
- at least one service alerting condition, the at least one service alerting condition being indicative a service operation to be immediately carried out by the user in order to maintain or restore operation of the ambulatory infusion system, the at least one service alerting condition having a medium alerting priority,
- at least one pre-service alerting condition, the at least one pre-service alerting condition being indicative for a upcoming future service alerting condition, the at least one pre-service alerting condition having a low alerting priority.

Typical examples for each of those alerting conditions will be discussed below in the context of exemplary embodiments.

The alerting unit may include any device for providing a noticeable alert to the device user, such as a visual indication, an audible indication, a tactile mechanical indication, such as a vibration, stimulation current generation, or the like. Deactivation is typically carried out by providing a confirmation command via an input unit, such as one or multiple push-buttons. As will be discussed below, an alerting status may also be automatically deactivated without requiring user input by the alert handling unit in some embodiments.

It should be noted that the deactivation is not necessarily associated with directly handling and resolving the situation that resulted in the alerting condition. In some cases, for example in case of an alarm clock function or reminder alerts, no dedicated handling may be required at all. In other cases, the occurrence of the alerting condition may be confirmed by the device user but actually dealt with later on. The phrase "dealing with an alerting condition" is used in the sense of removing the cause of its occurrence. For example, if an alert is provided due to the drug cartridge being empty, dealing with the alerting condition means replacing the drug cartridge.

In some embodiments, however, the ambulatory infusion system is configured to reactivate the same alerting status or to activate a different alerting status of typically higher alerting priority if the alerting condition is still present after confirmation. Some time delay may be foreseen after confirmation of an alerting status until the presence of the corresponding alerting condition is tested again. This means that the user does not receive an alarm again with respect to the same alerting status immediately after confirmation but has some time to actually deal with the alerting cause before he may be alerted again.

In some embodiments, the ambulatory infusion system is designed, while an alert with respect to an earlier alerting condition is provided, to detect the subsequent occurrence of a further alerting condition and to provide an alert wit respect to the earlier alerting condition or the further alerting condition in dependence of the corresponding alerting priorities. In this way, it is ensured that an alert is always provided with respect to the alerting condition of highest priority.

The corresponding steps may, for example be carried out every few milliseconds, every second, or every few minutes. It may further be synchronized with controlling the dosing unit to administer a drug dose which is typically carried out every few minutes. Different alerting conditions may be tested with the same or different frequencies.

In some embodiments, the alerting unit includes a visual indication unit and a non-visual indication unit, and an alert includes a visual alert and a non-visual alert. The visual indication unit is typically realized as display, but may, additionally or alternatively, be realized as flashlight, or the like. The non-visual indication unit typically includes an audio indicator, such as a loudspeaker or buzzer, and a tactile indicator, such as a pager vibrator. The indication unit may, besides providing alerts, be used as output unit of a general user interface for operating the system. Providing information with respect to the alerting condition may, for example be realized by displaying a numerical alert code, such as an error code and/or plain text information.

The alerting unit may be designed to provide information with respect to one alerting condition at the same time, but may also be designed to simultaneously provide information with respect to more than one alerting condition for which the corresponding alerting status is activated. The alerting unit, in particular a display, may, for example, show a list of alerting conditions which is currently activated. Such a list is favorably sorted according to alerting priorities and accordingly the order in which alerts will be provided with respect to the divers alerting conditions. The alerting condition having the highest alerting priority, that is, the alerting condition with respect to that an alert is currently being provided, is favorably always shown as top or bottom element of the list. Alternatively or additionally, the alerting condition of highest priority may be identified by different means, such as using a larger font, using a dedicated color, highlighting, or the like.

The indication unit may further be designed to provide an indication with respect to the alerting priority. This may be done, for example, via different display colors, symbols that are shown on the display, and the like.

The ambulatory infusion system may allow the device user to set some parameters with respect to the alert generation. In particular with respect to non-visual alerts, the user may select the level of audio alerts and/or whether alert indications shall be provided via audible indication, via tactile indication, or both. The ambulatory infusion system may be designed to vary the way of signalization in dependence of the alerting priority. It may, for example, be designed to override user settings in dependence of the alerting priority. For example, if the user selects to only receive tactile alerts, the ambulatory infusion device may be designed to provide additional audible alerts for alerting conditions with high alerting priority and/or to provide audible alerts of the highest possible volume even is the user selects a lower volume for audible alerts.

In some embodiments, the ambulatory infusion system is designed to operate alternatively in a standard mode or at least one special operation mode and to provide an alert with respect to an alerting condition that is detected while the ambulatory infusion system is operated in the special operation mode immediately or after return to the standard mode in dependence of the corresponding alerting priority.

The standard mode is an operation mode in which the ambulatory infusion system is typically operated most of the time as default mode. A typically present display may normally be switched off in the standard operation mode or provide information of general interest, such as time and/or date and the current basal infusion rate. Special operation modes that are different form the standard operation mode may be provided for various purposes. In particular, programming modes may be provided as special operation modes, for purposes such as setting the time and date, setting the basal infusion rate as a function of time and date and setting various therapy parameters. Further special operation modes may be provided for programming the infusion of drug boli according to a variety of profiles over time, for reviewing a typically present device history which stores, for example, information with respect past drug administrations and past alerting conditions, or one or multiple communication modes for data exchange with further devices, such as a PC. Typically, a special operation mode is entered by operating an input unit of a typically present user interface, for example a "Menu" push button. In dependence of the special operation mode, drug infusion may or may not be carried out in such a mode. The system may return to the standard operation mode via a dedicated user action and/or automatically, for example upon completion of a data exchange or upon a timeout event if no user operation occurs for a predetermined timeout period.

The ambulatory infusion system may in particular be designed such be designed such that alerts with a high alerting priority, as defined, for example by a priority threshold are provided in the special operation mode while alerts of low priority are provided only after return to the standard operation mode. For such an embodiment, it is ensured that high priority alerts are, for example alerts that require am immediate action, are provided without avoidable delay while operations that are carried out in a special operation mode may be continued and finished without disturbance or interruptions due to low priority alerts.

The ambulatory infusion system may be designed to change an alerting status with respect to which an alert is being provided to the deactivated state upon reception of a confirmation command by a user of the ambulatory infusion system.

In some of those embodiments, the confirmation command includes a first confirmation command and a further second confirmation command. The ambulatory infusion system is designed to stop the non-visual indication upon reception of the first confirmation command and to stop the visual indication upon reception of the second confirmation command. In such an embodiment, the generally disturbing non-visual indication may be stopped, e.g. by muting and audible alert, quickly and stopping the visual indication later on after reviewing the alert cause.

If an alert is confirmed, an alert with respect to the next subsequent alerting condition for which a corresponding alerting status is activated, is indicated in accordance with the alerting priorities.

In some embodiments, the ambulatory infusion system is designed to deactivate an alerting status upon reception of the second confirmation command.

In some embodiments, the ambulatory infusion system is designed to determine if a blocking time period is running and not to provide, within the blocking time period, an alert with respect to an alerting condition that occurs within the blocking time period. Blocking time periods are useful to prevent an alert generation under circumstances where it is particularly disturbing or undesired.

Blocking time periods, may for example be fixed time of days, for example during night time. Favorably such blocking time periods can be set by the user and are stored in a typically non-volatile memory, e.g. a configuration parameter memory, of the ambulatory infusion system.

Alternatively or additionally, a blocking interval may be defined at any time in order to prevent an alert generation, for example, during a business meeting, while visiting a concert, or the like.

In some of those embodiments, the ambulatory infusion system is designed to provide or not to provide, within a blocking time interval, an alert with respect to an alerting condition that is detected in the blocking time interval in dependence of the corresponding alerting priority. The system may especially be designed such that an alert is provided with respect to alert causes of high alerting priority, for example with an alerting priority being equal to or above a priority threshold, even if a blocking time interval is running. This allows informing the user with respect to an alert cause that requires immediate action without unnecessary delay while not unnecessarily disturbing where not required.

In some embodiments involving blocking time intervals, the ambulatory infusion system is designed to provide an alert with respect to an alerting condition that is detected in the blocking time interval after the blocking time interval having lapsed. This type of embodiment is especially useful for providing alerts that do not necessarily have to be provided as soon as possible according to their priority, but may also be provided with some additional delay, for example at the end of a business meeting. Typical examples for such alerts are alerts that inform the user about an upcoming handling operation, such as a cartridge replacement.

In some embodiments, the handling unit includes an alert queue, the alert queue being configured to store a number of activated alerting statuses in an order in which alerts shall be provided with respect to the activated alerting statuses. Alert queues are favorably realized by non-volatile memory in the control circuitry. The alert queue implementation may be in the form of one common alert queue for all alerting priorities. Alternatively, separate alert queues may be present for different alerting priorities. Exemplary alternative ways for implementing an alert queue are described in more detail below in the context of exemplary embodiments. Alternatively to an alert queue, the alert handling unit may include a set of flags, which each flag representing an alerting condition and a corresponding alerting status.

In some embodiments, the alert handling unit is designed, along with activating a new alerting status, to deactivate a previously activated alerting status, the new alerting status having the same or a higher alerting priority as compared to the previously activated alerting status.

This type of embodiments allows replacing a previously activated alerting status by a new one, which is especially helpful where an alert is provided to inform the user about an upcoming event at a time where immediate action is not required and a further alert is provided with respect to the same alert cause if the first alert is not reacted on in due time and is not confirmed. For example, the user may be informed via an alert about the remaining volume of the drug cartridge falling below a threshold level while a further alert is provided if the cartridge is actually empty. Here, the alert that indicates an empty drug cartridge may replace the alert indicating the low cartridge volume. Typically, the new alert has a higher alerting priority as compared to the previously activated alert. In some situations, however, the new alert may have the same alerting priority. This is the case, for example, if multiple reminders are provided with respect to an upcoming event. For example, several alerts of the same alerting priority may be provided to inform the user in time before the upcoming end of lifetime of the system.

In some embodiments, the ambulatory infusion system is designed to provide a secondary alert indication with respect to an alert cause, the secondary alert indication being further provided if the corresponding alerting status is deactivated. The secondary alert indication may be a visual indication, for example in form of a symbol that is shown on a device display. A secondary alert indication, such as, for example, a "Low Battery" indicator or a "Low Cartridge Volume" indicator is especially helpful to remind the user after confirmation of an alert, that the alert cause still needs to be handled.

In some embodiments, the ambulatory infusion system is designed to detect if an alerting cause has disappeared and to deactivate a previously activated alerting status with respect to that alert cause. In some cases, a device user may decide to immediately handle an alert cause, for example, by replacing a device battery, without having the alert previously confirmed. In such a situation, it is helpful for clarity as well as for convenience reasons that the corresponding alerting status is automatically cleared. Favorably, a potentially present secondary alert indication is cleared as well.

In some embodiments, the alerting unit includes a remote alerting unit, the remote alerting unit being physically separate from the dosing unit. In such an embodiment, the dosing unit and the remote alerting unit are designed to operatively couple such that upon detection of an alerting condition of the dosing unit the remote alerting unit is activated.

A remote alerting unit may especially be integral with a remote control device that may be present for controlling the system operation, and in particular the operation of the dosing unit, in everyday situations in a discrete add convenient way. The remote alerting unit may be the only alerting unit of the ambulatory infusion system or may be provided in addition to an alerting unit that is physically combined with the dosing unit, for example in a dosing device.

In embodiments where an alerting unit, for example a remote alerting unit, is used for indicating alerting conditions with respect to more than one physically separated devices, for example a remote control device and a dosing device, alerts favorably include information identifying the corresponding device, for example by displaying a device name.

In some embodiments, the ambulatory infusion system includes or is designed to operatively couple to an analyte concentration measuring system, wherein at least one of the number of alerting condition is an alerting condition of the analyte concentration measuring system. In the context of CSII, the analyte concentration may especially be a glucose meter, that is, a blood glucose measuring system. Such a blood glucose measuring system may be designed for carrying out single measurements at discrete points in time and be based, for example on an electro-chemical or opto-chemical principle, typically using single-use test strips in combination with a measuring device. Such a glucose meter may be integral with a remote control device or be provided as physically separate device. Alerting causes of the glucose meter may, for example, device errors, the occurrence of extremely high or extremely low measurement values, the use outside a temperature range in which reliable results can be expected, or the like.

In addition or alternatively, a continuous glucose measurement system may be provided that is designed to substantially continuously determine the user's blood glucose concentrations using a disposable subcutaneous or a long-term-implanted electrochemical sensor.

### Exemplary embodiments

In the following, exemplary embodiments are described in some more detail with reference to the figures.
Figure 1 a and Figure 1b shows an exemplary ambulatory infusion system in a schematic structural view and in a schematic perspective view, respectively.
Figure 2 shows typical alerting conditions for an ambulatory infusion system together with alert codes and alerting priorities.
Figure 3 shows an operational flow for detecting alerting conditions and providing alerts.
Figure 4 shows an operational flow for confirming alerts.
Figure 5, Figure 6 and Figure 7 illustrate alternative embodiments of an alert handling unit.
Figure 8a shows a further exemplary ambulatory infusion system in a schematic structural view.
Figure 8b shows an exemplary remote controller of an ambulatory infusion system in a schematic perspective view.

It should be noted that features which are described above in the context of the disclosure summary and which are not explicitly mentioned in the following description of exemplary embodiments, may be present in those embodiments as well. In addition, features that are only described in context of exemplary embodiments for clarity and conciseness reasons may be present in other alternative embodiments as well.

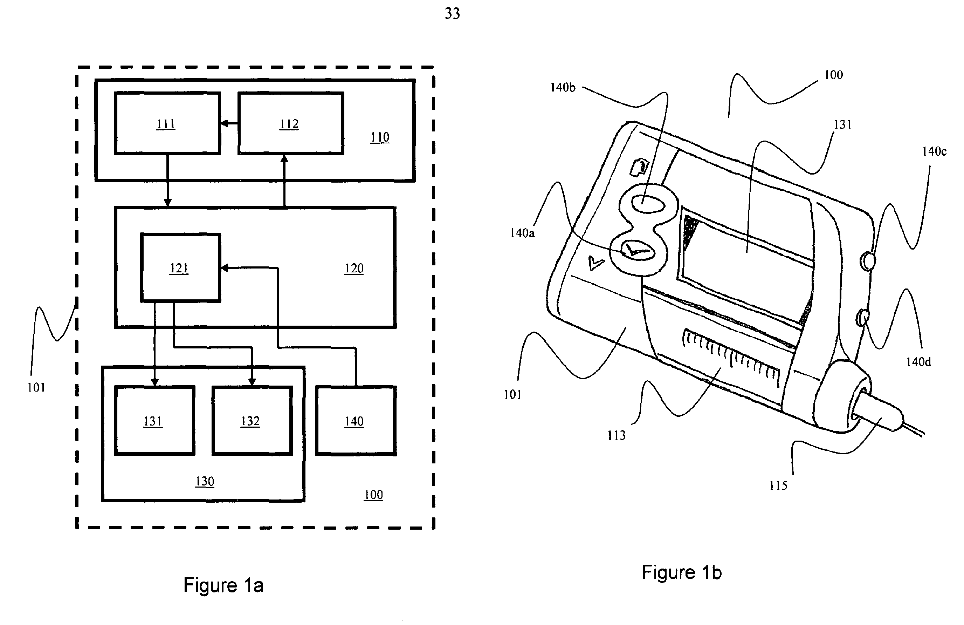

Figure 1 a shows an ambulatory infusion system 100 in accordance with the present disclosure in a schematic structural view. The ambulatory infusion system 100 includes a dosing unit 110 with an electronically controlled pump 112. The dosing unit 110 is exemplarily realized as syringe-driver as generally known in the art with the pump 112 comprising a motor-driven spindle drive. The dosing unit 110 further includes a sensor unit 111 which may, for example, include a motor encoder and a force sensor which determines the force exerted by the spindle drive of pump 112 onto a movable piston of a drug cartridge (not shown). The sensor unit 111 is used to monitor operation of the dosing unit 110 and to detect the presence of alerting conditions with respect to the dosing unit 110, such as the occurrence of a fluidic occlusion or a chemical blockage of the pump 112.

Alternatively, the dosing unit may be realized differently and include, for example, a peristaltic pump, a micro-membrane pump, or any kind of plunger-type pump.

The system further includes control circuitry 120. Control circuitry 120 typically includes components such as one or more microcontrollers running a corresponding firmware code, ASICS, memory components, discrete electronic circuitry, and the like. The control circuitry 120 in particular includes an alert handling unit 121 the operation of which is discussed below in more detail. The control circuitry may implements self-checking functionality in order to detect the presence of hardware errors, distortions of the system-internal communication, loss of memory data, and the like. The sensor unit 111 and the control circuitry 120, in combination, form a monitoring unit.

The control circuitry 120 controls the dosing unit 110 to infuse drug in a continuous or quasi-continuous way in accordance with a time-variable basal infusion profile. In case of CSII, this profile reflects the user's time-of-day dependent basal insulin demand. In addition, the control circuitry may control the dosing unit 110 to infuse a larger drug bolus on demand. An operation mode in which drug is infused is referred to as RUN mode. The control circuitry 120 may further control the ambulatory infusion system and in particular the dosing unit 110 to operate in at least one STOP or suspend mode where no drug is infused. Such a mode may be selected by a device user for certain maintenance or handling steps, such as replacing the drug cartridge. As will be discussed later on, the ambulatory infusion system 100 may further change into such a mode automatically upon occurrence of some alerting conditions.

The ambulatory infusion system 100 further includes an alerting unit 130, typically including a visual indication unit 131 and a non-visual indication unit 132. The visual indication unit 131 is typically realized as a display, such as a graphical LCD or OLED display. The non-visual indicating unit 132 typically includes an audio indicator, such as a buzzer or loudspeaker, a tactile indicator, such as a pager-vibrator, or a combination of both. The visual indication unit 131 is designed to show a clear text or coded information with respect to an alerting condition. The input unit 140 typically is realized by a number of push-buttons, but may also include a scroll-wheel, a touch-screen, or the like. In the context of the present disclosure, the input unit 140 is especially used for entering confirmation commands with respect to activated alerting statuses. However, the alerting unit 130 and the input unit 140, in combination, typically also form a general user interface of the ambulatory infusion system 100. The user interface is typically used for purposes such as programming drug amounts to be infused by the dosing unit 110, configuring the ambulatory infusion system 100, reviewing device history data that are typically stored in a non-volatile memory of the ambulatory infusion system, and the like.

The ambulatory infusion system 100 typically comprises further components that are not shown in Figure 1 for clarity purposes, such as a power supply in form of one or multiple rechargeable or non-rechargeable batteries. The components of the ambulatory infusion system 100 are enclosed by a typically water-tight housing 101 which may have a foot print comparable to a credit card and is about 1 - 2.5cm thick and is designed to be carried in trousers pockets, in a necklace-like way, with a belt clip, or the like. Alternatively or additionally, the housing 101 may be designed to be directly attached to a user's skin via an adhesive pad and/or be typically removable connectable to a cradle, such as a based plate which itself is attached to the users skin.

It should be noted that Figure 1 only shows functional components and operative couplings between those functional components that are of particular relevance with respect to the present disclosure.

Figure 1b shows the ambulatory infusion system 100 in an exemplary perspective view. From the alerting and output unit 130, the display 131, which for example an LCD dot-graphic display, is visible in Figure 1 b. The input unit 140 is realized by four pushbuttons 140a, 140b, 140c, 140d which are used for generally operating the system and for confirming alerts. Alerts may be confirmed for example by operating the "OK" button 140. Figure 1b also shows a drug container 113 in form of a cylindrical cartridge which is inserted into a container compartment (not referenced) in housing 101. A spindle (not referenced) of the pump 112 is arranged inline with the drug cartridge 113. The remaining filling volume of the drug container 113 is visible via a transparent cartridge window (not referenced). The drug cartridge is replaceable by the user. Figure 1b further shows a connector 115 which forms the upstream end of an infusion tubing (not referenced). The connector 115 may be a fluidic standard connector, such as a Luer connector, or a special purpose connector, e.g. a bayonet connector. The connector may further include components such as a drug flow sensor, a pressure sensor, a check valve, and the like. Alternatively, such components may be arranged as separate units between drug cartridge 113 and connector 115.

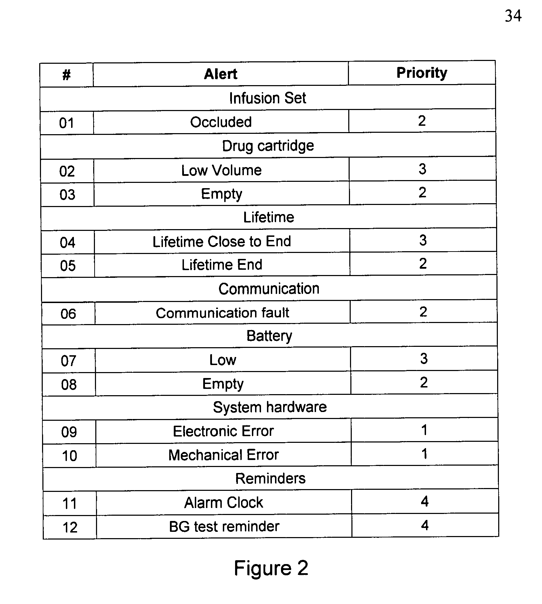

Figure 2a shows a table of alerting conditions that may occur in a device according to Figure 1. Each alerting condition is associated with a corresponding alerting status which may be activated and deactivated by the alert handling unit 121. For each alerting condition, the left colon of Figure 2 shows a numerical code while the middle colon shows corresponding clear texts. Upon an alerting condition being activated, the visual alerting unit 131 may show the numerical code, the clear text or both. The right colon of Figure 2 shows, for each alerting condition, the corresponding alerting priority. In the present example, four priorities 1 - 4 are present, with 1 being the highest and 4 being the lowest priority. It should be noted that the list of alerting conditions in Figure 2 may not be exhaustive, further alerting conditions may be present as well. Additionally or alternatively, some of the shown alerting conditions may not be present in a specific device. Similarly, the number of priority levels may be different.

The meaning of the exemplary alerting conditions of Figure 2a is as follows:
*#01 "Infusion Set: Occluded":* Under exceptional circumstances or if tubing and/or cannula are used for a too long time period without replacement, a fluidic blockage or occlusion may occur, such that such that infusion is not further possible. This situation is detected by sensor unit 111.
*#02 "Drug cartridge: Low volume":* This alerting condition occurs if the remaining drug volume in a drug cartridge has fallen below some low volume threshold. For a typical insulin cartridge as used in current ambulatory insulin pumps, having a total volume of about 300 IU (International Units), or 3ml of liquid insulin formulation having the concentration U100, the low volume threshold may, for example, be set to 20 IU or .2 ml. The low volume threshold may be a configurable parameter. The alert is provided to indicate to the device user that the drug container will soon be empty and has to be replaced in the next time.
*#03 "Drug cartridge: Empty":* This alerting condition occurs subsequent to alerting condition #03 and indicates that the drug cartridge is empty. It needs replacement before infusion can be continued.
*#04 "Lifetime: Close to End":* This alerting condition occurs some time before the ambulatory infusion system 100 approaches the end of its useful lifetime which is typically in the range of some years. An alert is favorably provided about 1 - 3 months before the actual end of lifetime. The phrase "End of lifetime" may refer to the absolute end of lifetime after which the ambulatory infusion system has to be discarded and replaced, or may be a usage time after which some service, maintenance, and/or check up is required, a leasing contract has to be renewed, or the like. Instead of a single alerting condition #05, a number of alerts may be provided at different remaining life times, for example six weeks, four weeks, and two weeks before the actual lifetime end.
*#05 "Lifetime: End":* This alerting condition indicates that the useful lifetime of the device has finally expired.
   The alerting conditions #04, #5 are highly are associated with the lifetime management and the corresponding safety policy and may especially not be present for a system of generally unlimited lifetime.
*#06 "Communication Fault":* In some embodiments, the ambulatory infusion system 100 includes one or multiple communication interfaces, such as an IR and/or RF interface for communicating with further devices, such as a glucose sensor, a remote control device, a personal computer, or the like. An alert is provided in case of a communication interruption, corrupted data transmission or the like.
*#07 "Battery Low":* This alerting condition occurs if a battery powering the ambulatory infusion system 100 is low and needs replacement and/or recharging within the next time, while the battery power is still sufficient for powering the system for some more time, for example, some hours or days.
*#08 "Battery Empty":* The battery is empty and needs replacement for continuing the infusion therapy.
*#09 "Electronic Error":* This error condition indicates the occurrence of some error within the electronic system, such as a component fault. For detecting the occurrence of electronic errors, typical ambulatory infusion systems carry out an extensive number of self-tests some of which are typically carried out with high frequency, for example every second or every few minutes, while others may be carried out, for example once per day. An electronic error may also be indicated if there is in fact no hardware error, but there is a sporadic problem for example in the device-internal communication.
*#10 "Mechanical error":* This error condition occurs in case of a mechanical fault, defective, or hazard of the pump 112, and in particular of its drive. Typically, the error is associated with a defect such that the system needs to be replaced or repaired.

The alerting conditions #01, #09, #10 are examples for malfunction alerts, the malfunction resulting either for a hazardous application situation, such as a clogged cannula, or a system defect.

The alerting conditions #03, #05, #08 are examples for service alerts since they are indicative for a situation where some service operation is required by the device user without which the operation can not be continued or restored.

The alerting conditions #02, #04, 07 are examples of pre-service alerts. A pre-service alerts indicates that a service operation will be required in the nearer future, without, however, requiring an immediate action.

The ambulatory infusion system 100 may additionally provide reminder alerts. Those reminders are provided for convenience purposes. They may, include, for example an alarm clock, (Alert #11) or a reminder for the user to test its blood glucose level (Alert #12), e.g., at a given time of day or some time after the administration of a drug bolus, e.g. an insulin bolus that is associated with a meal intake.

The behavior of an exemplary ambulatory infusion system upon occurrence of an alerting condition is typically different in dependence of the alerting condition, as summarized as follows:

| **Alerting condition** | **System behavior** |
|---|---|
| #11, #12 | Alert to be confirmed by user; Operation continued |
| #02, #04, #07 | Alert to be confirmed by user; Operation continued Secondary alert indication provided |
| #01, #03, #05, #06, #08, #10 | Alert to be confirmed by user; Change to STOP if in RUN |
| #09 | No confirmation of alert; Change to STOP if in RUN |

Generally, alerts that are provided as reminders or to inform the user of an upcoming situation requiring some user action, but do not need any steps to be taken immediately, may simply be acknowledged while the regular operation of the system continues. In particular, drug is further infused in the RUN mode. Dealing with the alerting condition results in the alerting status being deactivated if is has not been confirmed so far as well as the secondary alert indication being cleared.

For those alerts that indicate an upcoming user action, namely #02, #04, #07, a secondary alert indication is favorably continuously provided even after the alert has been confirmed, as long as the corresponding alerting condition is present. The secondary alert indications may be provided in form of a message or symbol that is indicated via the visual indicator 631, such as a "Low cartridge" symbol or a "Low Battery" symbol.

Alerting conditions that do not allow the drug infusion to be continued result in the system to switch into a STOP or suspend mode which is maintained after the alert being confirmed. In most cases, the device user may, having dealt with the alerting condition, switch the system back into the RUN mode. Dealing with the situation, may, for example, be the replacement of the drug cartridge for alerting condition #03 (Drug Cartridge Empty), or replacing the infusion cannula for alerting condition #01 (Infusion Set occluded).

As exception, an electronic error #09 can favorably not be simply confirmed and the alert indication can only be stopped by removing the battery powering the system. In this situation, the user may try to deal with the alerting condition by removing and subsequently re-inserting the battery after a waiting period, thus powering-up the system. If this approach is not successful, a defect is typically present.

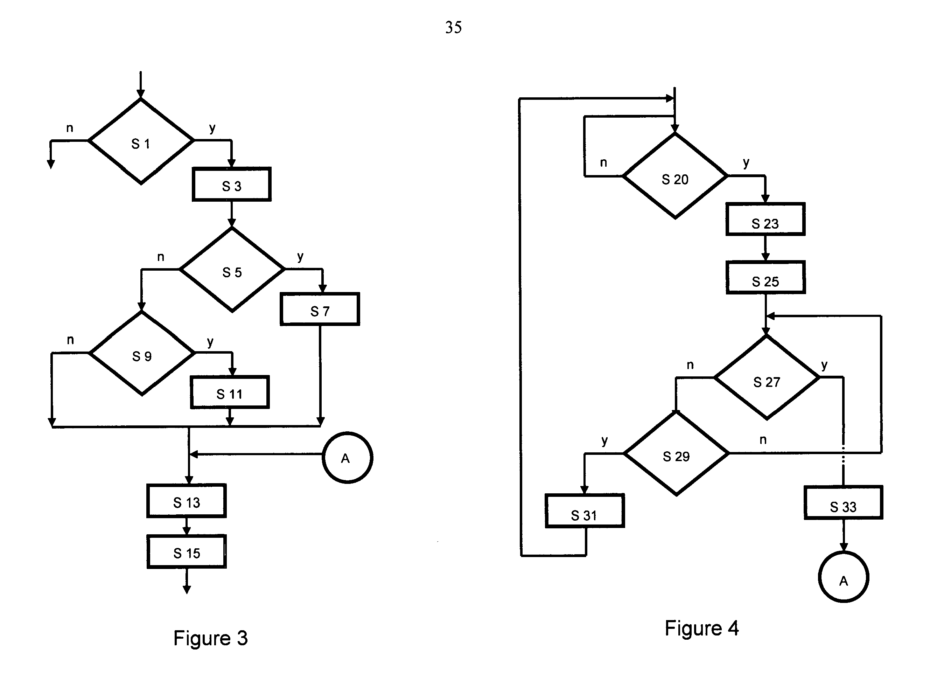

Figure 3 shows an operational flow for detecting alerting conditions and providing corresponding alerts in a schematic view. The operational flow as shown in Figure 3 is carried out in a substantially continuous way during operation of the ambulatory infusion system.

In step S 1, the occurrence of an alerting condition is tested. If no alerting condition has occurred, the normal operational flow is continued. If an alerting condition has occurred, the operational flow proceeds with the step S 3 where the corresponding alerting status is activated. In step S 5, it is tested whether the alerting condition should result in a mode change of the ambulatory infusion system, in particular a change into a STOP or suspend mode as explained above. If a mode change is required, it is carried out in step S 7.

For alerting conditions which do not require a mode change, it is tested in step S 9 whether a secondary alert indication should be provided to the user as explained above. If this is the case, the corresponding secondary alert indication is provided in step S 11, for example by activating a corresponding symbol of visual indicator 31.

In step S 13, the activated alerting statuses, if any, are searched for an alerting status of the highest priority as will be discussed below in more detail.

In step S 15, the alerting unit is controlled to provide an alert with respect to the alerting condition determined in step S 13.

In embodiments including the consideration of blocking time intervals, step S13 may include, following the determination of an activated alerting status of the highest alerting priority, if a blocking time interval is running. If no blocking time interval is running, the operational flow may proceed as shown. If a blocking time interval is running, step S15 may be carried out immediately if the alerting priority of the alerting condition as detected in step S1 equals or exceeds a priority threshold and be carried out only after the blocking time interval has lapsed otherwise.

Similarly, if the ambulatory infusion system 100 may operate in a standard operation mode and at least one special operation mode as described above, the operational flow as shown in Figure 3 refers to an operation in the standard operation mode. If the system operates in a special operation mode, Step S 15 may be carried out immediately if the alerting priority of the alerting condition as detected in step S1 equals or exceeds a priority threshold and be carried out only after return to the standard operation mode otherwise.

For the for the exemplary four priority levels of Figure 2, an appropriate priority threshold may, for example, be 1 or 2.

Figure 4 shows an exemplary operational flow for confirming alerts in a schematic view. Like the operational flow of Figure 3, the operational flow as shown in Figure 4 is carried out substantially continuously during operation of the ambulatory infusion system.

In step S 20, the entry of a first confirmation command by the user is being awaited. The first confirmation command may be provided, for example, by operating a dedicated button of input unit 140, for example an "OK" button. Upon reception of the first confirmation command by the alert handling unit 121, the non-visual alert indication is deactivated in step S 23.

In subsequent step S 25, a re-signalization timer is started. In step S 27, the reception of a secondary confirmation command is being tested. The secondary confirmation command may be provided, for example, by operating the same button as for the first confirmation command a second time. If no second confirmation command is provided in step S 27, the re-signalization timer is tested in S 29 to determine if a re-signalization threshold time has expired. If this is the case, the non-visual indication is activated again in step S 31 and the operational flow proceeds again with awaiting a first confirmation signal in step S 20.

If the re-signalization threshold time has not expired, the operational flow continues with awaiting the second confirmation command in step S 27.

If the second confirmation command is being provided, the alerting status is deactivated in step S 33 and the visual alert indication is cleared. Since alerting statuses having the same or a lower alerting priority may still be active, operational flow subsequently proceeds with step S 13 of Figure 3.

The confirmation by a first and second confirmation command, in combination with the re-signalization timer, ensures that the user can deactivate or mute the generally disturbing tactile and/or audible signalization quickly but does not forget about the alerting condition.

It should be noted that the operational flow as explained and shown in Figure 3 and Figure 4 is somewhat simplified for clarity reasons. In addition, some steps may be omitted, such as the provision of secondary alerting indicators, and/or single steps may be carried out in different order. Page 17, line 22: In addition, the order of some operational steps may be changed. For example, it would be possible to activate a secondary alert indication (step S 9) only after the first or second confirmation command.

The operational flow is carried out under control of alert handling unit 121.

In the following, alternative exemplary ways for the operation of alert handling unit 121 is described in more detail by way of example situations.

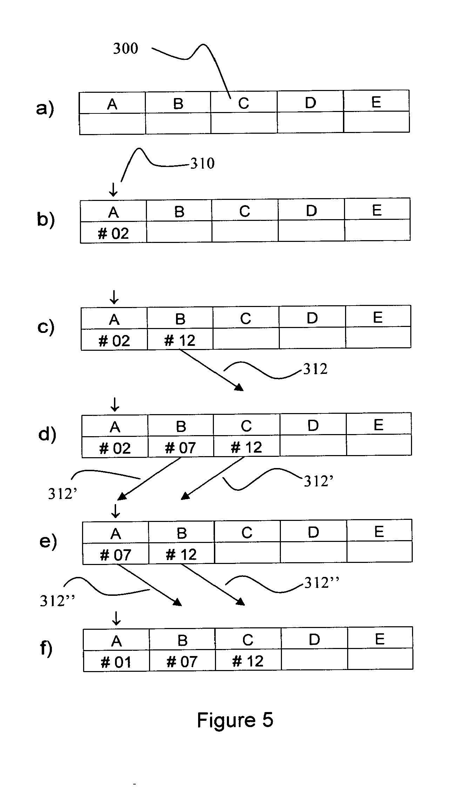

Figure 5 illustrates an embodiment where the alert handling unit 121 includes a alert queue 300 that is shared for all alerting statuses and stores the activated alerting statuses in an order given by their alerting priorities.

The alert queue 300 is typically realized as a set of memory registers that are identified by corresponding labels "A" to "E". Each register may store a code corresponding to an activated alerting status. The alert queue is favorably realized as non-volatile memory, such that information with respect to activated alerting statuses is not lost in case of a power supply interruption, e.g. when replacing a battery. The alert queue 300 is organized such that an alert with respect to the alerting condition of register "A" is provided first and with respect to the alerting condition of register "E" last. Consequently, the alerting status for which an alert is being provided, if any, is always stored in register "A".

It shall be noted that the number of 5 registers as shown in Figure 5 is exemplary, the number may be higher or lower. In general, an appropriate number of registers is given by a number of alerting statuses that may be simultaneously active.

In the following, the relative position of the registered is described with "left" and right", with register "A" being the leftmost and register "E" being the rightmost register. That is, the alerting order is from left to right.

Operation of the alert handling unit 121 for this type of embodiment may be summarized as follows. If, starting from an empty alert queue 300, an alerting status is activated, it is stored in register "A" of the alert queue and a corresponding alert is provided. Providing an alert is generally indicated by the presence of arrow 310, pointing towards register "A". If the ambulatory infusion system is designed to simultaneously provide information with respect to more than one alerting condition, arrow 310 indicates the alerting condition of highest priority which should be explicitly indicated, as described above.

If a further alerting status is activated with the alert queue 300 not being empty, the further alerting status is inserted into the alert queue 300 in accordance with its alerting priority. That is, it is inserted such that all registers to the left store alerting statuses having the same or a higher priority while all registers to the right store alerting statuses having the same or a lower alerting priority.

Alerting statuses having the same alerting priority may be dealt with in different way. In particular, they may be inserted left or right of other alerting statuses having the same alerting priority. In the first case, an alert with respect to the most recent alerting condition is provided first while the alerting conditions are indicated in accordance with their order of occurrence in the latter case. The latter case is assumed in the following. It should be noted that it is also possible to assign a unique alerting priority for each alerting status. For such an embodiment, the order in which alerts are provided is clearly defined independent of their order of occurrence.

When inserting an alerting status, the original content of the corresponding register as well as the content of all registers right of this register is right-shifted by one position.

When the alerting status of register "A" is deactivated, the contents of all registers of the alert queue 300 is left-shifted by one position. Thereby, the confirmed alerting status is removed from the alerting queue 300 and the next following alerting status is stored in register "A" and the corresponding alert is accordingly provided and/or indicated as alert of the highest priority to be confirmed next.

In the following, an exemplary sequence of situations is described with reference to Fig. 5a to 5f. Numerical error codes are used for both identifying alerting conditions as well as the corresponding alerting statuses.

Figure 5a shows a situation where no alerting status is active, registers "A" to "E" are accordingly empty. Starting from an empty alert queue, Figure 5b indicates a situation after occurrence of alerting condition #02, "Drug Cartridge: Low Volume".

Since no further alerting status is activated, an alert is provided with respect to that alerting condition, as indicated by arrow 310. No immediate action is required by the user for settling the alerting condition, regular operation and in particular drug infusion continues.

Figure 5c indicates a subsequent situation where an additional alerting condition #12 "BG test reminder" has been activated without the alerting status #02 in register "A" having been deactivated. As shown in Figure 2, alerting condition #12 has a lower priority as compared to alerting condition #02. Therefore, register "A" is not changed and #12 is stored in register "B".

Figure 5d illustrates a still subsequent situation where an additional alerting status #07 "Battery Low" has been activated without the already present alerting statuses having been deactivated. Alerting status #07 has the same alerting priority as alerting status #02. It is stored in register "B", that is, behind register "B" and the original content of register "B" right-shifted, as indicated by arrow 312.

Figure 5e shows a still subsequent situation after the alerting status #02 having been deactivated by confirming it (step S 33 in Figure 4). Confirming the alerting status, however, does not necessarily mean that the alerting cause has to dealt with and settled.

Along with deactivating alerting status #02, all registers of the alerting queue 300 are left-shifted by one position, as indicated by arrows 312'. This situation is illustrated in Figure 5e. Now, an alert is provided with respect to alerting status #07 as new content of register "A".

Figure 5f illustrates a still subsequent situation after occurrence of an infusion set occlusion and activation of alerting status #01 without the activated alerting statuses #07, #12 having been deactivated. Because alerting condition #01 has a higher alerting priority as compared to the already activated alerting statuses, it is entered in register "A" of the alert queue such that the corresponding alert is immediately provided, with the already present register contents being right-shifted by one position, as indicated by arrows 312".

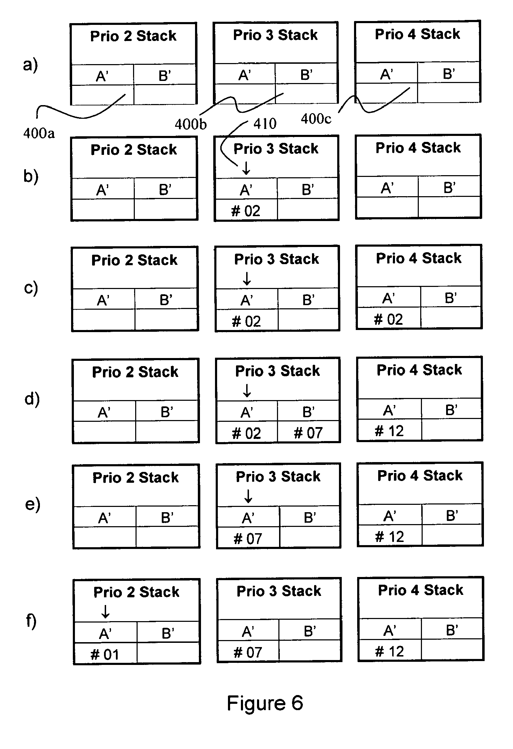

Figure 6 a) to f) show the same situation as Figure 5 a) to f) for an alternative embodiment of alert handling unit 121. In contrast to the embodiment illustrated in Figure 5, a separate alert queue is provided for each alerting priority. Figure 6 shows alerting queues 400a, 400b, and 400c for alerting priorities 2, 3, and 4 respectively. An alert queue for alerting priority 1 is also present in the alert handling unit 121 but is not shown in Figure 6 because no alert of priority 1 occurs in the illustrative example. The single alert queues 400a, 400b, 400c are exemplarily shown with two registers A', B' each.

Upon an alerting status being activated, it is entered to the corresponding alerting queue in accordance with its alerting priority as given in Figure 2. For each alerting priority, the organization and management of the corresponding alert queue is carried out in the same way as described above with reference to Figure 5.

For the embodiment Figure 6, alerts are provided as follows: The non-empty alert queue of highest priority is determined, that is, the alerting queue of highest priority for which register A' as leftmost register is not empty. The Alert is provided according to the alerting status stored in that register. In Figure 5 a) to f), this register is marked by arrow 410.

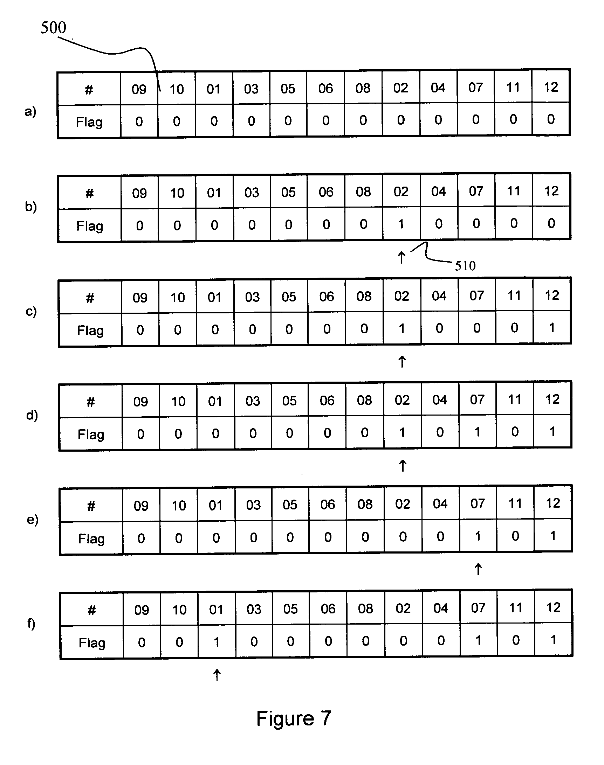

Figure 7 a) to f) show the same situation as Figure 5 a) to f) for another alternative embodiment of alert handling unit 121. In contrast to the embodiments as discussed before, no dedicated alert queue is present in this embodiment. Instead, a binary set of flags 500 is provided, with each flag corresponding to one alerting condition and corresponding alert status. In Figure 7a) to e) the alerting conditions are given in the upper row of each table while the status of the flags is given by the lower row. Each flag may either be set or reset. A set flag reflects an activated alerting status and is indicated by a "1" in the corresponding cell. A reset flag reflects a deactivating alerting status and is indicated by a "0" in the corresponding cell. Like in the previously described embodiments, the set of flags 500 is favorably realized by non-volatile memory.

The set of flags 500 is arranged such that the flags are sorted according to their alerting priority, the alerting priority descending from left to right. In this way, the alerting priorities of Figure 2 do not need to be explicitly stored but are given implicitly via the order of the flags.

For this type of embodiment, the activated alerting condition of the highest priority with respect to which an alert is accordingly provided is given by the leftmost flag that is set. In Fig. 7, the corresponding flag is marked by arrow 510.

It is, of course, also possible to arrange the flags in a different order which does not correspond to the alerting priorities. In this case, the alerting priorities are explicitly stored.

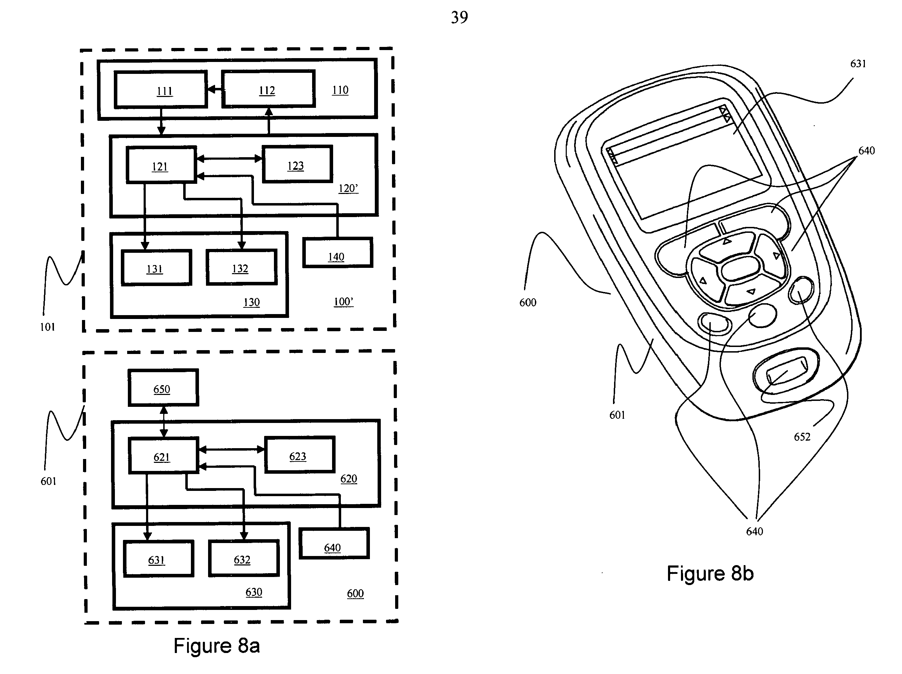

Figure 8 shows an ambulatory infusion system 100', 600 according to a further exemplary embodiment. Many functional components as well as their interaction is identical or substantially identical with those of Figure 1, with identical or substantially identical functional components having identical reference numbers. The following description is focused on the differences.

The ambulatory infusion system according to this type of embodiment includes a dosing device 100' and a remote control device 600, the two devices being physically separated and having different housings 101 and 601, respectively. The remote control device 600 is favorably a discreet and small handheld device, having a shape and size comparable to a cell phone, a pocket calculator, a TV remote controller, a PDA (Personal Digital Assistant), or the like. It is also possible to realize the remote control device 600 integral with any of such devices.

The remote control device 600 includes control circuitry 620, with control circuitry 620 including a remote control alert handling unit 621. Control Circuitry 620 further includes a communication interface 623 that is designed to operatively couple with a corresponding communication interface 123 of dosing device 100', favorably via short-range RF communication, e.g. according to the Bluetooth standard. The remote control device 600 further includes an output unit 630 with visual indicator 631 and one or more non-visual indicators 632 as well as an input unit 640. The indicators 631, 632 of the output unit 630 serve as alert indicators as well as for general user output and feedback purposes. The input unit 640 serves for entering confirmation commands. The output unit 630 and the input unit 640, in combination, additionally serve as user interface for operating the remote control device 600 and for remotely controlling the dosing device 100'.

The remote control device 600 further includes an optional blood glucose measurement system 650 that is operatively coupled to the control circuitry 620. The blood glucose measurement system may be of any appropriate design known in the art, and be, based, for example on en opto-chemical or electro-chemical principle and use either single one-use test strips or include a cassette or the like with a plurality of test fields.

The remote control alert handling unit 621 generally operates in the same way as the alert handling unit 121 of dosing device 100' and may especially handle alerting conditions of different alerting priorities in an analogue way.

The alert handling unit 121 of the dosing device 100' is intended for handling alerting conditions of the dosing device 100' while the remote control alert handling unit 621 of the remote control device 600 is designed to handle alerts of both the dosing device 100' and the remote control device 600 as follows.

Alerts with respect to an alerting condition of the remote control device 600, such as, for example, a low or empty battery of the remote control device 600, an electronics error of the remote control device 600, or an invalid blood glucose measurement by measurement system 650 are provided on the remote control device only and are dealt with in an analogous ways as described above.

Activated alerting statuses of the dosing device 100' are transmitted to the remote control device 600 via communication interfaces 123, 623 and are indicated via the indicators 631, 632, of the remote control device. In one embodiment, a communication channel between dosing device 100' and remote control device 600 is automatically established upon occurrence of an alerting condition of the dosing device 100'. Alternatively, a communication channel may be established and activated alerting statuses may be transmitted only upon the user switching on the remote control device 600'.

In one variant, alerts with respect to alerting conditions of the dosing device 100' are additionally be provided via the alerting unit 130 of the dosing device 100'. Alternatively, they are only provided on the remote control device 600 if a communication channel is established. In embodiments where the remote control device 600 may provide alerts with respect to both the dosing device 100 - as well as the remote control device 600, it favorably provides a device identification along with an alert.

Confirmation commands with respect to alerting conditions of the remote control device 600 are entered via the input unit 640 of the remote control device, while confirmation commands with respect to alerting conditions of the dosing device 100' may favorably alternatively be entered via either of input units 140, 640, respectively.

In a variant, no input unit 140 is present in the dosing device 100'. This may especially be the case for embodiments of the dosing device 100' that are designed to be carried in a patch-like way directly at the infusion site. It is in principle further possible to omit the alerting unit 130 of the dosing device 100 and to only relay on the output unit 630 of the remote control device 600. At least a non-visual indicator, such as a buzzer, loudspeaker or pager vibrator, however, is favorably provided in the dosing device 100' for safety and redundancy reasons.

In a still further variant, the alert handling unit 121 is not present and all alert handling is carried out on the remote control alert handling unit 621.

In a still further variant, a continuous glucose monitoring system, based, for example on a subcutaneous electrochemical sensor or micro-dialysis sensor is present for continuously or quasi-continuously monitoring the user's blood glucose level. Such a system may be provided alternatively or in addition to the glucose measurement system 650 and may be designed for wired or wireless communication with the remote control device 600. Alerting conditions of the continuous glucose monitoring system may be detected and handled in an analogous way as alerting conditions of the dosing unit 100' and the remote control device 600, respectively.

Figure 8b shows an exemplary perspective view of a handheld remote control device 600. Form output unit 630, a display 631 is visible which is exemplarily realized as graphical color LCD display. The input unit 640 is realized by a number of push buttons. One or more of them may be used for alert confirmation purposes. A test strip slot 6520 of blood glucose measurement system 650 is provided for inserting single electro-chemical blood glucose test strips.

## Claims

1. Ambulatory infusion system (100, 100', 600), the ambulatory infusion system (100, 100', 600), including:
(a) an electronically controlled dosing unit (110), the dosing unit (110) being designed to be carried by a user for an extended time period and concealed from view,
(b) a monitoring unit (111, 120, 620), the monitoring unit (111, 120, 620) being designed to repeatedly test the occurrence of a number of alerting conditions of the ambulatory infusion system (100, 100', 620) and to change, upon detection of an alerting condition, a corresponding alerting status from a deactivated state to an activated state while maintaining other alerting statuses unaffected,
(c) an alerting unit (130, 630), the alerting unit (130, 630) being designed to provide an alert to a device user with respect to an alerting condition, the alert including information identifying the alerting condition;
(d) an alert handling unit (121, 621), the alert handling unit (121, 621) storing an alerting priority for each of the alerting conditions and being designed to repeatedly determine, among the activated alerting statuses, an alerting status of the highest alerting priority and to control the alerting unit (130, 630) to provide an alert with respect to the corresponding alerting condition.

2. Ambulatory infusion system (100, 100', 600) according to Claim 1, wherein the ambulatory infusion system (100, 100', 600) is configured, while an alert with respect to an earlier alerting condition is provided, to detect the subsequent occurrence of a further alerting condition and to provide an alert with respect to the earlier alerting condition or the further alerting condition in dependence of the corresponding alerting priorities.

3. Ambulatory infusion system (100, 100', 600) according to either of the preceding claims, wherein the alerting unit (130, 630) includes a visual indication unit (131, 631) and a non-visual indication unit (132, 632), and wherein an alert includes a visual alert and a non-visual alert.

4. Ambulatory infusion system (100, 100', 600) according to either of the preceding claims, wherein the ambulatory infusion system (100, 100', 600) is designed to claims, wherein the ambulatory infusion system (100, 100', 600) is designed to change an alerting status with respect to which an alert is being provided to the deactivated state upon reception of a confirmation command by a user of the ambulatory infusion system (100, 100', 600).

5. Ambulatory infusion system (100, 100', 600) according to Claim 3 and Claim 4, wherein the confirmation command includes a first confirmation command and a further second confirmation command, and wherein the ambulatory infusion system (100, 100', 600) is designed to stop the non-visual indication upon reception of the first confirmation command and to stop the visual indication upon reception of the second confirmation command.

6. Ambulatory infusion system (100, 100', 600) according to Claim 5, wherein the ambulatory infusion system (100, 100', 600) is designed to change an alerting status to the deactivated state upon reception of the second confirmation command.

7. Ambulatory infusion system (100, 100', 600) according to either of the preceding claims, wherein the alert handling unit (121, 621) is designed to store at least three different alerting priorities.

8. Ambulatory infusion system (100, 100', 600) according to Claim 7, wherein the number of alerting conditions includes
• at least one malfunction alerting condition, the at least one malfunction alerting condition being indicative for a malfunction of the ambulatory infusion system, the at least one malfunction alerting condition having a high alerting priority,
• at least one service alerting condition, the at least one service alerting condition being indicative a service operation to be immediately carried out by the user in order to maintain or restore operation of the ambulatory infusion system (100, 100', 600), the at least one service alerting condition having a medium alerting priority,
• at least one pre-service alerting condition, the at least one pre-service alerting condition being indicative for a upcoming future service alerting condition, the at least one pre-service alerting condition having a low alerting priority.

9. Ambulatory infusion system (100, 100', 600) according to either of the preceding claims, wherein the ambulatory infusion system is designed to determine if a blocking time period is running and not to provide, within the blocking time period, an alert with respect to an alerting condition that occurs within the blocking time period.

10. Ambulatory infusion system (100, 100', 600) according to either of the preceding claims, wherein the ambulatory infusion system is designed to operate alternatively in a standard mode or at least one special operation mode and to provide an alert with respect to an alerting condition that is detected while the ambulatory infusion system is operated in the special operation mode immediately or after return to the standard mode in dependence of the corresponding alerting priority.

11. Ambulatory infusion system (100, 100', 600) according to either of the preceding claims, wherein the alert handling unit (131, 631) includes an alert queue (400, 500), the alert queue (400, 500) being configured to store a number of activated alerting statuses in an order in which alerts shall be provided with respect to the activated alerting statuses.

12. Ambulatory infusion system (100, 100', 600) according to either of the preceding claims, the wherein monitoring unit (111, 120, 620) includes a sensor unit (111), the sensor unit (100) being operatively coupled to the dosing unit (100) to monitor a physical characteristic, the physical characteristic reflecting an occurrence of an alerting condition of the dosing unit (110).

13. Ambulatory infusion system (100, 100', 600) according to either of the preceding claims, wherein the alert handling unit (100, 100', 600) is designed to detect if an alerting cause has disappeared and to deactivate a previously activated alerting status with respect to that alert cause.

14. Ambulatory infusion system (100', 600) according to either of the preceding claims, wherein the alerting unit (130, 630) includes a remote alerting unit (630) the remote alerting unit (630) being physically separate from the dosing unit (110), and wherein the dosing unit (110) and the remote alerting unit (630) are designed to operatively couple such that upon detection of an alerting condition of the dosing unit (110) the remote alerting unit (630) is activated.

15. Ambulatory infusion system (100', 600) according either of the preceding claims, the ambulatory infusion system (100', 600) including or being designed to operatively couple to an analyte concentration measuring system (650), wherein at least one of the number of alerting condition is an alerting condition of the analyte concentration measuring system (650).
